# EUROPEAN PATENT SPECIFICATION

(11) **EP 4 324 394 B1**
(45) Date of publication and mention of the grant of the patent: **16.09.2026**
(21) Application number: 22782804.3
(22) Date of filing: 21.03.2022
(51) Int. Cl.: A61B 5/1495, A61B 5/145, A61B 5/155, A61B 5/00, G16H 50/20

(54) **METHOD FOR CALIBRATING NON-INVASIVE BIOMETRIC INFORMATION**
VERFAHREN ZUR KALIBRIERUNG NICHTINVASIVER BIOMETRISCHER INFORMATIONEN
MÉTHODE D'ÉTALONNAGE D'INFORMATIONS BIOMÉTRIQUES NON INVASIVES

(30) Priority: 20.05.2021 KR 20210065023
(43) Date of publication of application: 21.02.2024
(73) Proprietor: i-Sens, Inc., Seoul 06646 (KR)
(72) Inventor: KANG, Young Jea, Seoul 06646 (KR); NAM, Hak Hyun, Seoul 06646 (KR)
(74) Representative: BCKIP Part mbB
(86) International application number: PCT/KR2022/003887
(87) International publication number: WO 2022/244963

(56) References cited:
- CN-A- 107 438 398
- JP-A- 2009 039 263
- JP-A- 2009 039 267
- JP-A- 2011 062 335
- KR-A- 20200 075 512
- KR-A- 20210 017 482
- US-A1- 2019 110 751
- US-B2- 10 687 739

## Description

### TECHNICAL FIELD

The present invention relates to a method for calibrating non-invasive biometric information measured by a non-invasive biometric information measurement device, and more specifically, by learning non-invasive biometric information measured by a non-invasive blood glucose meter with continuous biometric information measured by a continuous blood glucose meter, the non-invasive biometric information can be calibrated so as to be personalized to a user, and by using the continuous biometric information measured by the continuous blood glucose meter to calibrate non-invasive biometric information measured by the non-invasive blood glucose meter so as to be personalized to the user, the pattern of increase and/or decrease in user biometric information can be accurately determined from the still uncertain non-invasive biometric information.

### BACKGROUND

Diabetes is a chronic medical condition that is common in modern people, and in the Republic of Korea, there are 2 million diabetes patients, about 5% of the total population.

Diabetes occurs when the absolute level of the glucose level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Diabetes requires constant blood glucose measurement for management, so the demand for devices related to blood glucose measurement is steadily increasing. Various studies have confirmed that in the cases of diabetic patients who strictly control their blood glucose level, the incidence of diabetes complications is significantly reduced. Accordingly, it is very important for diabetic patients to measure blood glucose regularly to control blood glucose levels.

The finger prick method is generally used to manage blood glucose in diabetic patients. These blood glucose meters help diabetic patients manage their blood glucose, but since they only show results at the time of measurement, it is difficult to accurately determine frequently changing blood glucose levels. In addition, these blood glucose meters require blood sample collection to measure blood glucose levels frequently throughout the day, which poses a significant burden on diabetic patients.

To overcome such a drawback, continuous glucose monitoring systems (CGMSs) inserted into the human body to measure a blood glucose level every few minutes have been developed, and therefore easily perform the management of diabetics and responses to an emergency situation.

A continuous glucose monitoring system is a device that extracts interstitial fluid with a sensor partly inserted into a body of a user and measures a blood glucose level in real time from the extracted body fluid. A sensor for a continuous glucose monitoring system that is partially inserted into a human body extracts body fluids while being inserted into the body for a certain period of time, for example, approximately 15 days, and the continuous glucose monitoring system periodically generates blood glucose biometric information from the extracted interstitial fluids and provides it to the user.

However, a continuous glucose monitoring system has also inconvenience of having to replace a sensor every 1 week, 15 days, or 1 month and insert the sensor into the body, and moreover, there is the inconvenience of having to use a separate blood glucose meter at each set time to calibrate the measured blood glucose level.

Meanwhile, recently, non-invasive blood glucose meters that can measure blood glucose levels with a portable device without collecting blood are being researched and developed. Non-invasive blood glucose testing is harmless to the human body, painless, has no side effects, and has excellent test reproducibility. It is the blood glucose testing method that all diabetic patients dream of.

Research to noninvasively measure blood glucose has been conducted steadily since the 1990s, and approaches using various principles are being attempted.

Non-invasive blood glucose measurement methods being researched and developed can be broadly divided into four principles. The initially attempted method was to measure blood glucose in subcutaneous tissue by attaching a consumable patch to the skin (transdermal), but measurement techniques limited to thin skin layers have limitations in accuracy and reproducibility. After this, a method of determining blood glucose concentration was attempted by using optical principles to incident light and measure and analyze the spectrum of the reflected light, and much research is still being conducted in this field. Meanwhile, some methods have been attempted to measure blood glucose by providing electrical stimulation and inducing an electro-chemical reaction (Electrochemical), or methods of measuring blood glucose concentration using ultrasonic waves that penetrate deeply into the living body have been proposed (Ultrasound).

However, non-invasive blood glucose meters have the advantage of being able to measure blood glucose painlessly from outside the skin or by contacting the skin, but it is more inaccurate than the existing methods of measuring blood glucose by collecting blood directly from the body or by extracting body fluid, additionally, because the physical or physiological conditions of each user are different, there is a problem that it is difficult to accurately measure blood glucose in the same way for all users.

US 2019 / 0 110 751 A1 discloses an apparatus for providing corrected bio-information by using a bio-information sensor. The apparatus includes a communicator configured to receive bio-information from the bio-information sensor, a processor configured to extract metabolic information based on food intake information of a user and correct the received bio-information based on the extracted metabolic information, and an outputter configured to provide a result of correcting the bio-information.

US 10 687 739 B2 discloses an apparatus for monitoring blood glucose comprising an invasive component for invasively measuring blood glucose and a non-invasive component, including color image sensor(s) to generate images from absorption of light that traversed the tissue, to receive a body part and generate a non-invasive blood glucose reading. Processors convert the images into a vector associated with a particular at least one invasive blood glucose measurement, form a regular learning matrix, implement a noninvasive isolation mechanism of the tissue glucose level by unique association of the vector with an invasive blood glucose level, determine a neural network from the learning set by pairing vectors into a branch and forming multiple branches into loops, wherein two vectors are paired if they have a pre-defined similarity in the blood glucose levels that each are associated with, and calibrate the neural network by having it pass at least one test.

### SUMMARY

### Technical Problem

The present invention is to solve problems in conventional non-invasive biometric information measurement methods mentioned above, and the purpose of the present invention is to learn non-invasive biometric information measured by a non-invasive blood glucose meter with continuous biometric information measured by a continuous blood glucose meter and provide a method to calibrate non-invasive biometric information so as to be personalized to a user.

Another purpose of the present invention is to provide a method for calibrating non-invasive biometric information that can accurately determine even increase and/or decrease patterns in the biometric information of a user from non-invasive biometric information by using continuous biometric information measured by a continuous blood glucose meter to calibrate non-invasive biometric information measured by a non-invasive blood glucose meter so as to be personalized to a user.

Still another purpose of the present invention is to provide a method for calibrating non-invasive biometric information that can accurately determine the biometric information of a user using only event information and non-invasive biometric information without continuous biometric information by comparing non-invasive biometric information and continuous biometric information according to the entered event information to personalize and learn continuous biometric information corresponding to the non-invasive biometric information when an event occurs.

Still another purpose of the present invention is to provide a method for calibrating non-invasive biometric information that can judge or predict the biometric information of a user using only the event information and the non-invasive biometric information and provide an alarm to the user in an emergency situation by comparing the non-invasive biometric information and the continuous biometric information according to the entered event information to personalize and learn continuous biometric information corresponding to the non-invasive biometric information when an event occurs.

### Solution to Problem

To accomplish the purpose of the present invention, the present invention provides a method in accordance with claim 1.

A method of calibrating non-invasive biometric information according to the present invention comprises: measuring continuous biometric information of a user by a continuous biometric information measurement device in which a part of a sensor is inserted into a body of the user and measures biometric information of the user over a certain period of time; measuring non-invasive biometric information of the user for the certain period of time using a non-invasive biometric information measurement device that measures the biometric information of the user at a distance away from or in contact with skin of the user; and learning the continuous biometric information corresponding to the non-invasive biometric information to the user by comparing the continuous biometric information with the non-invasive biometric information measured for the certain period of time.

The method of calibrating the non-invasive biometric information according to an embodiment of the present invention further comprises, when additional non-invasive biometric information of the user is acquired by the non-invasive biometric information measurement device after the certain period of time, calibrating the additional non-invasive biometric information by determining continuous biometric information learned to correspond to the additional non-invasive biometric information.

By comparing an increase and/or decrease pattern of the non-invasive biometric information with an increase and/or decrease pattern of the continuous biometric information, the increase and/or decrease pattern of the continuous biometric information corresponding to the increase and/or decrease pattern of the non-invasive biometric information may be personalized to the user and learned, and by determining the increase and/or decrease pattern of the continuous biometric information learned to correspond to the increase and/or decrease pattern of the additional non-invasive biometric information from the additional non-invasive biometric information, an increase and/or decrease pattern of the additional non-invasive biometric information may be calibrated.

The method of calibrating the non-invasive biometric information according to an embodiment of the present invention further comprisse acquiring event information about events that occur to the user during the certain period of time, wherein by comparing the continuous biometric information and the non-invasive biometric information according to the event information, the continuous biometric information corresponding to the non-invasive biometric information may be personalized to the user and learned when the events occur.

Here, the event information may be input through a displayed interface screen for event input.

The method of calibrating the non-invasive biometric information further comprises, when acquiring the event information, acquiring occurrence condition information of the events occurred to the user together, wherein, by comparing the continuous biometric information and the non-invasive biometric information according to the event information and the occurrence condition information, the continuous biometric information corresponding to the non-invasive biometric information is personalized to the user and learned when the events occur with the occurrence condition information.

Here, the occurrence condition information may be at least one of season information, time information, place information, location information, temperature information, and humidity information when the events occur.

Here, the occurrence condition information may be input through a displayed interface screen for occurrence condition input.

Here, in the method of calibrating the non-invasive biometric information, continuous biometric information of the user is measured over multiple certain periods of time by a plurality of continuous biometric information measurement devices, and by comparing continuous biometric information and non-invasive biometric information measured over the multiple certain periods of time, continuous biometric information corresponding to the non-invasive biometric information for the multiple certain periods of time is personalized to the user and learned.

Here, the multiple certain periods of time are set to be apart from each other, as one example, the multiple certain periods of time are set to be apart from each other at a same time interval, and as another example, the multiple certain periods are set to be apart by at least one of an environmental condition, a seasonal condition, a user physical condition, and a user physiological condition.

Preferably, the method of calibrating the non-invasive biometric information according to one embodiment of the present invention may further comprise, if later event information occurred to the user after the certain period of time and additional non-invasive biometric information of the user are acquired, calibrating the additional non-invasive biometric information by determining continuous biometric information learned to correspond to the later event information and the additional non-invasive biometric information.

Preferably, the method of calibrating the non-invasive biometric information according to another embodiment of the present invention may further comprise, if later event information occurred to the user after the certain period of time, occurrence condition information of an later event which the later event occurs, and additional non-invasive biometric information of the user are acquired, calibrating the additional non-invasive biometric information by determining continuous biometric information learned to correspond to the later event information, the occurrence condition information of the later event, and the additional non-invasive biometric information.

In the method of calibrating the non-invasive biometric information according to an embodiment of the present invention, by comparing an increase and/or decrease pattern of the non-invasive biometric information with an increase and/or decrease pattern of the continuous biometric information, the increase and/or decrease pattern of the continuous biometric information corresponding to the increase and/or decrease pattern of the non-invasive biometric information is personalized to the user and learned, and by determining the increase and/or decrease pattern of additional non-invasive biometric information from the additional non-invasive biometric information and an increase and/or decrease pattern of continuous biometric information learned to correspond to the additional event information, the increase and/or decrease pattern of the additional non-invasive biometric information is calibrated.

According to the present invention, the method of calibrating non-invasive biometric information may comprises: measuring continuous biometric information of a user by a continuous biometric information measurement device in which a part of a sensor is inserted into a body of the user and measures biometric information of the user over a certain period of time; measuring non-invasive biometric information of the user for the certain period of time using a non-invasive biometric information measurement device that measures the biometric information of the user at a distance away from or in contact with skin of the user; and acquiring event information about an event occurred to the user while measuring the continuous biometric information; by comparing the event information and an increase and/or decrease pattern of the non-invasive biometric information with an increase and/or decrease pattern of the continuous biometric information, learning by personalizing the increase and/or decrease pattern of the continuous biometric information corresponding to the event information and the increase and/or decrease pattern of the non-invasive biometric information to the user; acquiring additional non-invasive biometric information of the user and additional event information occurred to the user by the non-invasive biometric information measurement device after the certain period of time; and by determining the increase and/or decrease pattern of continuous biometric information learned to correspond to an increase and/or decrease pattern of the additional non-invasive biometric information determined from the additional non-invasive biometric information and the additional event information, calibrating the increase and/or decrease pattern of the additional non-invasive biometric information.

Here, the event information and the additional event information may be input through a displayed interface screen for event input.

The method of calibrating the non-invasive biometric information further comprises, when acquiring the event information, acquiring occurrence condition information of the event occurred to the user together, wherein, by comparing the increase and/or decrease pattern of the non-invasive biometric information according to the event information and the occurrence condition information with the increase and/or decrease pattern of the continuous biometric information, the increase and/or decrease pattern of the continuous biometric information corresponding to the increase and/or decrease pattern of the non-invasive biometric information is personalized to the user and learned when the event occurs with the occurrence condition information.

Here, the occurrence condition information of the event or occurrence condition information of an additional event is input through a displayed interface screen for occurrence condition input.

Preferably, the method of calibrating the non-invasive biometric information according to still another embodiment of the present invention may further comprise: determining an increase and/or decrease change rate based on the calibrated increase and/or decrease pattern of the additional non-invasive biometric information; and when the determined increase and/or decrease change rate exceeds a threshold change rate, providing an alarm to the user.

Preferably, the method of calibrating the non-invasive biometric information according to still another embodiment of the present invention may further comprise: predicting a future increase and/or decrease change rate based on the calibrated increase and/or decrease pattern of the additional non-invasive biometric information; and when the predicted future increase and/or decrease change rate exceeds a threshold change rate, providing an alarm to the user.

### Advantageous Effects of Invention

A method for calibrating non-invasive biometric information according to the present invention has the following effects.

First, the method for calibrating non-invasive biometric information according to the present invention learns non-invasive biometric information measured by a non-invasive biometric information measurement device with accurate continuous biometric information measured by a continuous biometric information measurement device, so that non-invasive biometric information can be calibrated so as to be personalized to a user even if the physical and biological conditions are different for each user.

Second, the method for calibrating non-invasive biometric information according to the present invention calibrates the non-invasive biometric information measured by the non-invasive biometric information measurement device so as to be personalized to a user using continuous biometric information measured by the continuous biometric information measurement device, so that even an increase and/or decrease pattern of biometric information can be accurately determined from the non-invasive biometric information that is still inaccurate.

Third, the method for calibrating non-invasive biometric information according to the present invention compares the non-invasive biometric information and the continuous biometric information according to the input event information, and personalizes and learns continuous biometric information corresponding to the non-invasive biometric information when an event occurs, so that afterwards, even without the continuous biometric information, the biometric information of a user can be accurately determined using only the event information and the non-invasive biometric information.

Fourth, the method for calibrating non-invasive biometric information according to the present invention compares the non-invasive biometric information and the continuous biometric information according to the input event information, and personalizes and learns continuous biometric information corresponding to the non-invasive biometric information to the user when an event occurs, so that the biometric information of a user can be determined or predicted using only the event information and the non-invasive biometric information and an alarm can be provided to the user in an emergency situation.

### BRIEF DESCRIPTION OF THE DRAWINGS

FIG. 1 is a diagram for schematically illustrating a non-invasive biometric information calibration system for use with a method according to an embodiment of the present invention.
FIG. 2 is a diagram illustrating a non-invasive biometric information calibration system for use with a method according to another embodiment of the present invention.
FIG. 3 is a functional block diagram for explaining a non-invasive biometric information calibration device for use with a method according to the present invention.
FIG. 4 is a diagram for explaining the operation of the learning unit for use with a method according to the present invention.
FIG. 5 is a diagram for explaining the operation of the calibration unit for use with a method according to the present invention.
FIG. 6 is a flowchart illustrating a non-invasive biometric information calibration method according to an example not covered by the present invention.
FIG. 7 is a flowchart illustrating a non-invasive biometric information calibration method according to an embodiment of the present invention.
FIG. 8 shows an example of non-invasive blood glucose information measured by a non-invasive biometric information measurement device and continuous blood glucose information measured by a continuous biometric information measurement device.
FIG. 9 explains a certain period of time during which a continuous biometric information measurement device is worn on a body.
FIG. 10 illustrates an example of an interface screen for inputting event information in the present invention.
FIG. 11 illustrates an example of event information input through an input interface screen.
FIG. 12 is a diagram illustrating an example of displaying blood glucose information measured using only a non-invasive biometric information meter after removing the continuous biometric information meter.
FIG. 13 is a flowchart for explain an example of providing an alarm to a user based on an increase and/or decrease pattern of blood glucose information.
FIG. 14 is a flowchart illustrating an example of providing an alarm to a user based on a subsequent increase and/or decrease change rate of blood glucose information.
FIG. 15 shows an example of an alarm message provided to a user.

### DETAILED DESCRIPTION OF EMBODIMENTS

In the present disclosure, it should be noted that the technical terms are only used to describe specific embodiments and are not intended to limit the present invention. In addition, the technical terms used in the present disclosure, unless specifically defined differently in the present invention, should be interpreted as meanings generally understood by a person skilled in the art to which the present invention pertains.

Additionally, singular expressions used in the present invention include plural expressions unless the context clearly dictates otherwise. In the present disclosure, terms such as "consists of" or "comprises" should not be interpreted as necessarily including all of the various components or steps described in the disclosure, and it should be interpreted that some of the components or steps may not be included, or additional components or steps may be included.

In addition, it should be noted that the attached drawings are only intended to facilitate easy understanding of the concepts of the present invention and should not be construed as limiting the present invention by the attached drawings.

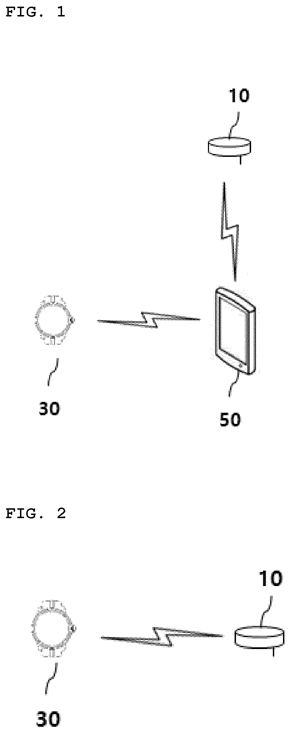

FIG. 1 is a diagram for schematically illustrating a non-invasive biometric information calibration system according to an example, and FIG. 2 is a diagram illustrating a non-invasive biometric information calibration system according to another example.

The non-invasive biometric information calibration system include a continuous biometric information measurement device (10) and a non-invasive biometric information measurement device (30), and can calibrate non-invasive biometric information with a separate user terminal (50) or can directly calibrate non-invasive biometric information from the non-invasive biometric information measurement device (30) without the separate user terminal (50).

Hereinafter, the continuous biometric information measurement device (10) and the non-invasive biometric information measurement device (30) will be respectively described as measuring a blood glucose level of a user, but depending on the field to which the present invention is applied, the continuous biometric information measurement device (10) and the non-invasive biometric information measurement device (30) can be used as devices that can measure various biometric information.

First, looking at the system for calibrating non-invasive biometric information through the user terminal (50) with reference to FIG. 1, the continuous biometric information measurement device (10) is equipped with a sensor, and part of the sensor is inserted and attached to the body of a user for a certain period of time to measure a blood glucose information of a user by extracting body fluids from the body, and the non-invasive biometric information measurement device (30) is a device that can measure the blood glucose information of a user in a non-invasive manner by contacting the skin of a user or away from the skin while worn by the user.

The user terminal (50) is connected to the continuous biometric information measurement device (10) in a wireless or wired manner and receives the continuous blood glucose information of the user measured periodically or upon request from the continuous biometric information measurement device (10), and the user terminal (50) is connected in a wireless or wired manner to the non-invasive biometric information measurement device (30) and receives the non-invasive blood glucose information of the user measured periodically or upon request from the non-invasive biometric information measurement device (30).

Preferably, the user can input information about events that occurred to the user into the user terminal (50) during a certain period of time while the continuous biometric information measurement device (10) is attached. An interface screen for inputting event information is displayed on the user terminal (50), and the user can input the event information that will occur later or the event information that previously occurred on the interface screen. Depending on the field to which the present invention is applied, a multi-level sensor may be further included to detect events occurring to the user, and for example, the user terminal (50) may determine an event occurring in the user by an activity sensor, a location sensor, etc., and automatically input the determined event upon user confirmation.

Here, the event may affect the blood glucose level of the user. For example, the event may be an event that increases the blood glucose level, such as when the user eats breakfast, lunch, dinner, or a snack, or an event that reduces the blood glucose level, such as exercising, working, studying, etc. In order to more accurately determine the effect of an event on the blood glucose level of the user, detailed event information about the type of food consumed by the user, the amount of food consumed, etc. is entered together, or details event information about the type of exercise performed by the user, exercise time, etc. may be entered together.

Preferably, when an event occurs, the user can additionally input information about the event occurrence conditions into the user terminal (50). An interface screen for inputting the information about the event occurrence conditions is displayed on the user terminal (50), and the user can input the information about the event occurrence conditions through the interface screen. Depending on the field to which the present invention is applied, a multi-level sensor may be further included to detect the occurrence conditions of an event that occurs to the user. For example, the user terminal (50) may determine the information about the event occurrence conditions by a location sensor, temperature sensor, humidity sensor, etc., or may obtain occurrence condition information through a network. The information about the event occurrence conditions can be automatically entered by user confirmation.

The user terminal (50) is equipped with a storage means that can store continuous blood glucose information, non-invasive blood glucose information, event information, and occurrence condition information for a certain period of time, and also equipped with a processor means that can personalize and learn continuous blood glucose information corresponding to the non-invasive blood glucose information from continuous blood glucose information, non-invasive blood glucose information, event information, and occurrence condition information using a learning model stored in the storage means,

And the user terminal (50) receives continuous blood glucose information from the continuous biometric information meter (10) and receives non-invasive blood glucose information from the non-invasive biometric information meter (30), and by comparing the non-invasive blood glucose information with the continuous blood glucose information over a certain period of time, the continuous blood glucose information corresponding to the non-invasive blood glucose information can be personalized and learned for the user.

Preferably, in addition to continuous blood glucose information and non-invasive blood glucose information, the user terminal (50) acquires information on events that occur in the user over a certain period of time and information on conditions for occurrence when an event occurs, and by comparing non-invasive blood glucose information and continuous blood glucose information according to the event information and the information about occurrence conditions, using the information about occurrence conditions, continuous blood glucose information corresponding to the non-invasive blood glucose information can be personalized and learned for the user when an event occurs.

That is, using the continuous biometric information measurement device (10) and the user terminal (50), the continuous biometric information measurement device (10) continuously measures the blood glucose information for a certain period of time, for example, 1 week, 15 days, or 1 month, and the user terminal (50) can personalize and learn continuous blood glucose information corresponding to non-invasive blood glucose information for the user using continuous blood glucose information measured over a certain period of time.

The continuous biometric information measurement device (10) is removed after being attached to the body of the user for a certain period of time, and the blood glucose information is determined using only the non-invasive biometric information measurement device (30), and when the user terminal (50) receives non-invasive blood glucose information using a calibration model generated as a result of learning, the non-invasive blood glucose information measured by the non-invasive biometric information measurement device (30) is calibrated by applying the non-invasive blood glucose information, event information, and the information about event occurrence conditions to the calibration model.

Through this, when measuring blood glucose information using only a conventional non-invasive biometric information meter, the disadvantages of blood glucose information being inaccurate or different blood glucose values being measured for each user are overcome, and non-invasive biometric information is personalized to the user to accurately measure blood glucose information. Or at least, it is possible to accurately determine the user's blood glucose increase and/or decrease pattern. Through this, it is possible to overcome the disadvantages that when measuring a blood glucose information using only a conventional non-invasive biometric information meter, the blood glucose information is inaccurate or different blood glucose values are measured for each user, so by personalizing non-invasive biometric information to the user, it is possible to accurately measure blood glucose information or at least accurately determine the blood glucose increase and/or decrease pattern.

Next, looking at the system for non-invasively calibrating biometric information without the mediation of the user terminal (50) with reference to FIG. 2, the continuous biometric information measurement device (10) has a part of the sensor inserted and attached to the body of a user to extract body fluid for a certain period of time to measure blood glucose information, and The non-invasive biometric information measurement device (30) measures the blood glucose information in a non-invasive manner while the user wears it for a certain period of time.

The continuous biometric information measurement device (10) and the non-invasive biometric information measurement device (30) are communicationally connected in a wireless or wired manner, and the non-invasive biometric information measurement device (30) receives the continuous blood glucose information measured periodically or upon request from the continuous biometric information measurement device (10).

Preferably, the user can input information about events that occurred to the user into the non-invasive biometric information measurement device (30) during a certain period of time while the continuous biometric information measurement device (10) is attached. An interface screen for inputting event information is displayed on the non-invasive biometric information measurement device (30), and the user can input event information that will occur later or event information that previously occurred through the interface screen. Depending on the field to which the present invention is applied, multi-level sensors may be further included to detect events occurring in the user, and for example, the non-invasive biometric information measurement device (30) may determine an event that occurred to the user by an activity sensor, a location sensor, etc., and automatically input the determined event upon user confirmation.

Preferably, when an event occurs, the user can additionally input information about the event occurrence conditions into the non-invasive biometric information measurement device (30). An interface screen for entering the information about event occurrence conditions is displayed on the non-invasive biometric information measurement device (30), and the user can input the information about event occurrence conditions on the interface screen. Depending on the field to which the present invention is applied, multi-level sensors may be further included to detect the occurrence conditions of events that occur to the user. For example, the non-invasive biometric information measurement device (30) can determine the information about event occurrence conditions by a location sensor, temperature sensor, humidity sensor, etc., or obtain occurrence condition information through a network. The information about event occurrence conditions can be automatically entered upon user confirmation.

The non-invasive biometric information measurement device (30) is equipped with a storage means that can store continuous blood glucose information, non-invasive blood glucose information, event information, and occurrence condition information for a certain period of time, and also equipped with a processor means that can personalize and learn continuous blood glucose information corresponding to the non-invasive blood glucose information from continuous blood glucose information, non-invasive blood glucose information, event information, and occurrence condition information using a learning model stored in the storage means,

And the non-invasive biometric information measurement device (30) can compare the continuous blood glucose information received from the continuous biometric information measurement device (10) with the non-invasive blood glucose information and the continuous blood glucose information corresponding to the non-invasive blood glucose information can be personalized and learned for the user.

Preferably, the non-invasive biometric information measurement device (30) can personalize and learn continuous blood glucose information corresponding to non-invasive blood glucose information using occurrence condition information when an event occurs, by comparing non-invasive blood glucose information and continuous blood glucose information according to the event information and occurrence condition information.

That is, by using the continuous biometric information measurement device (10) together with the non-invasive biometric information measurement device (30), the continuous biometric information measurement device (10) may measure the continuous blood glucose level of the user for a certain period of time, for example, 1 week, 15 days, or 1 month, and the non-invasive biometric information measurement device (30) can personalize and learn continuous blood glucose information corresponding to the non-invasive blood glucose information to the user using continuous blood glucose information measured over a certain period of time.

The continuous biometric information measurement device (10) is removed after being attached to the body of the user for a certain period of time, and the blood glucose information is measured using only the non-invasive biometric information measurement device (30), and in the case that the non-invasive biometric information measurement device (30) acquires non-invasive blood glucose information using a calibration model generated as a result of learning, the non-invasive blood glucose information, the event information, and the information of event occurrence conditions are applied to the calibration model to correct the non-invasive blood glucose information.

Through this, by overcoming the disadvantages that the blood glucose information is inaccurate or the blood glucose value measured is different for each user when measuring blood glucose information using only a conventional non-invasive biometric information meter, it is possible to accurately measure blood glucose information or at least accurately determine a blood glucose increase and/or decrease pattern of a user by personalizing non-invasive biometric information to the user.

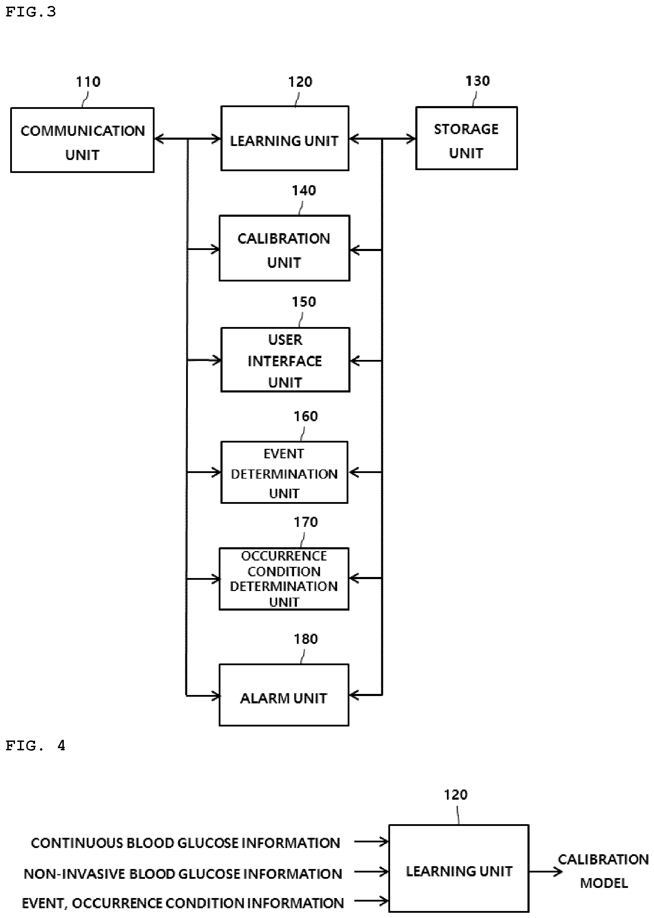

FIG. 3 is a functional block diagram for explaining a non-invasive biometric information calibration device. The non-invasive biometric information calibration device described in FIG. 3 can be implemented as a user terminal in the case of FIG. 1 and as a non-invasive biometric information measurement device in the case of FIG. 2.

Looking more specifically with reference to FIG. 3, the communication unit (110) communicates with an external terminal and transmits and receives data. Here, in the case that the non-invasive biometric information calibration device is implemented as a user terminal, the communication unit (110) transmits and receives data with a continuous biometric information measurement device and a non-invasive biometric information measurement device, and in the case that the non-invasive biometric information calibration device is implemented in a non-invasive biometric information measurement device, the communication unit (110) transmits and receives data with a continuous biometric information measurement device. The communication unit (110) can transmit and receive data with an external terminal in a wired or wireless manner. For example, data can be transmitted and received using methods such as Bluetooth, NFC (Near Field Communication), infrared communication, Wi-Fi communication, and USB cable communication.

A continuous biometric information measurement device is attached to a body of a user and continuously measures continuous blood glucose information over a certain period of time, and a non-invasive biometric information meter is worn on the user to measure blood glucose information in a non-invasive manner. A storage unit (130) stores measured continuous blood glucose information and non-invasive blood glucose information. A learning unit (120) applies continuous blood glucose information measured by a continuous biometric information meter over a certain period of time and non-invasive blood glucose information measured by a non-invasive biometric information meter to the learning model, and the continuous blood glucose information corresponding to the non-invasive blood glucose information is personalized to the user and learned.

Preferably, it is possible to obtain event information that occurred to the user while continuously measuring blood glucose information for a certain period of time or information about occurrence conditions of a event at the time the event occurred, and the storage unit (130) stores the acquired continuous blood glucose information, non-invasive blood glucose information, event information, and information about event occurrence conditions. When the learning unit (120) uses the continuous the blood glucose information to personalize and learn the continuous blood glucose information corresponding to the non-invasive blood glucose information for the user, using the continuous blood glucose information, the non-invasive blood glucose information, the event information, and the information about event occurrence condition stored in the storage unit (130), the non-invasive blood glucose information and the continuous blood glucose information according to the event information, and the occurrence condition information are compared, and using the occurrence condition information, the continuous blood glucose information corresponding to the non-invasive blood glucose information when the event occurs can be personalized and learned for the user.

That is, the learning unit (120) uses a training data set consisting of the continuous blood glucose information, the non-invasive blood glucose information, the event information that occurred while measuring the continuous blood glucose information, and the information about event occurrence conditions when the event occurs, and when an event occurs, the continuous blood glucose information corresponding to the non-invasive blood glucose information is personalized to the user and learned using the information about occurrence conditions, and a calibration model is generated to calibrate the non-invasive blood glucose information from the learning results.

Here, the learning unit (120) may perform learning using various learning model algorithms, such as generalized linear models (GLM), decision trees, random forests, gradient boosting machine (GBM), and deep learning. Depending on the field to which the present invention is applied, various learning model algorithms can be used when learning continuous blood glucose information corresponding to non-invasive blood glucose information personalized to the user.

Here, event information can be directly input by a user through an interface screen for inputting events displayed on the user interface unit (150), and information about event occurrence conditions can be directly input by the user through an interface screen for inputting occurrence conditions displayed on the user interface unit (150).

Depending on the field to which the present invention is applied, the event that occurred to the user can be determined by the event determination unit (160), and the event determination unit (160) may determine an event that occurred to the user based on information received from an activity sensor, a location sensor, etc. Preferably, when the event determination unit (160) determines the occurred event, the information about the determined event is output to the user interface unit (150), and when confirmation is received from the user, it is confirmed as an event that occurred to the user.

When an event occurs, information about event occurrence conditions can be determined by an occurrence condition determination unit (170) according to the field to which the present invention is applied, and the occurrence condition determination unit (170) can determine information about event occurrence conditions based on information received from a location sensor, activity sensor, temperature sensor, humidity sensor, etc., or season information, place information, location information, temperature information, and humidity information obtained through a network. Preferably, when the occurrence condition determination unit (170) determines the information about event occurrence conditions, the occurrence condition information of the determined event is output to the user interface unit (150), and when confirmation is received from the user, the occurrence condition information of the event is confirmed.

A continuous biometric meter is used to create a personalized calibration model for non-invasive blood glucose information, and after the calibration model is created, it is removed from the body of a user. The calibration unit (140) calibrates the non-invasive blood glucose information by applying the non-invasive blood glucose information measured by the non-invasive biometric information measurement device to the calibration model. Preferably, when the event information, information about event occurrence conditions, and non-invasive blood glucose information that occur in the user are obtained after the continuous biometric information measurement device is removed, non-invasive blood glucose information is calibrated by applying event information, information about event occurrence conditions, and non-invasive biometric information to the calibration model.

The alarm unit (180) determines the increase and/or decrease change rate of the non-invasive blood glucose information from the calibrated non-invasive blood glucose information, and when the increase and/or decrease change rate of the non-invasive blood glucose information exceeds the critical change rate, an alarm is provided to the user, or the future increase and/or decrease change rate of the non-invasive blood glucose information is predicted from the calibrated non-invasive blood glucose information, and when the future increase and/or decrease change rate of non-invasive blood glucose information exceeds the threshold change rate, an alarm is provided to the user.

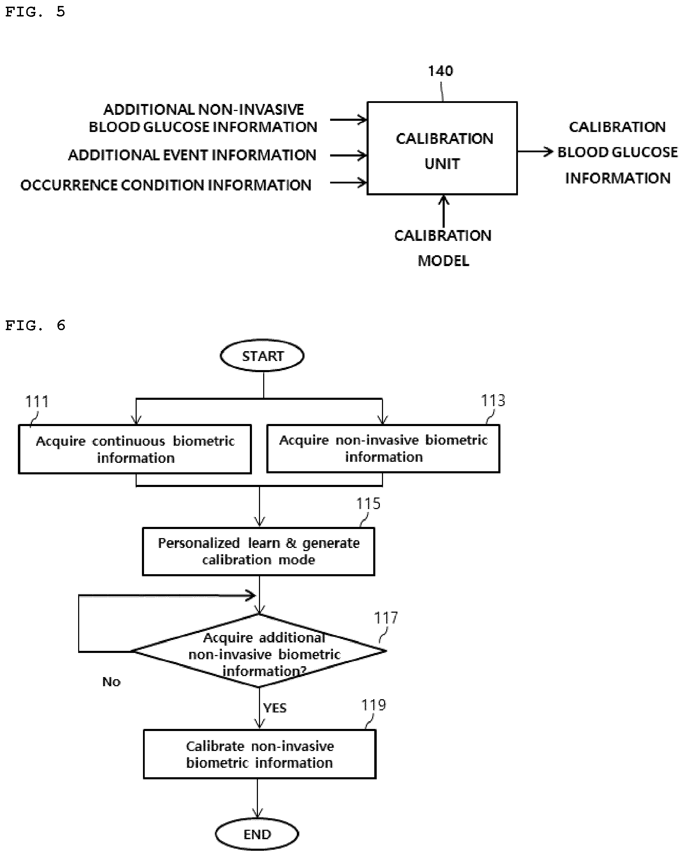

FIG. 4 is a diagram for explaining the operation of the learning unit, and FIG. 5 is a diagram for explaining the operation of the calibration unit.

Firstly, looking at the operation of the learning unit with reference to FIG. 4, when continuous blood glucose information and non-invasive blood glucose information are input to the learning unit (120), the learning unit (120) applies the non-invasive blood glucose information and continuous blood glucose information of the same time to model algorithm, then, continuous blood glucose information corresponding to the non-invasive blood glucose information is personalized to the user and learned, and a calibration model for calibrating the non-invasive blood glucose information is created as a result of the learning.

Preferably, in addition to continuous blood glucose information and non-invasive blood glucose information, event information and information about occurrence conditions when an event occurs may be input to the learning unit (120), and the learning unit (120) applies continuous blood glucose information, non-invasive blood glucose information, event information, and information about occurrence conditions to a learning model algorithm to personalize and learn continuous blood glucose information corresponding to the non-invasive blood glucose information with occurrence condition information when an event occurs, and as a result of learning, a calibration model is created to calibrate non-invasive blood glucose information.

Meanwhile, looking at the operation of the calibration unit with reference to FIG. 5, after the continuous biometric information measurement device is removed from the body, the blood glucose information of the user is measured using only the non-invasive biometric information measurement device. The calibration unit (140) can calibrate the additional non-invasive blood glucose information by applying the additional non-invasive blood glucose information obtained after the continuous biometric information meter is removed to the calibration model.

Preferably, in addition to the non-invasive blood glucose information, the calibration unit (140) may input additional event information and additional occurrence condition information when an additional event occurs, and the calibration unit (140) can calibrate additional non-invasive blood glucose information by applying non-invasive blood glucose information, event information, and information about event occurrence conditions to the calibration model.

FIG. 6 is a flowchart illustrating a non-invasive biometric information calibration method according to an example not covered by the present invention.

Looking more specifically with reference to FIG. 6, when continuous blood glucose information is measured by a continuous biometric information meter for a certain period of time, continuous blood glucose information is received and obtained (S111), and when non-invasive blood glucose information is measured by a non-invasive biometric information measurement device for a certain period of time, non-invasive blood glucose information is acquired (S113).

A continuous biometric information measurement device is attached to a body for a certain period of time to continuously measure blood glucose information and is removed from the body after a certain period of time, and continuous blood glucose information measured over a certain period of time and continuous blood glucose information over a certain period of time and non-invasive blood glucose information measured at the same time are applied to the learning model algorithm to perform personalized learning for the user and generate a calibration model from the learning results (S115).

By comparing non-invasive blood glucose information and continuous blood glucose information measured at the same time, features of the non-invasive blood glucose information are extracted, and continuous blood glucose information corresponding to the extracted features is learned to create a calibration model, or a calibration model can be created by applying the non-invasive blood glucose information and continuous blood glucose information measured at the same time to the input node of the artificial neural network model and calculating the weight of the hidden node using a linear regression method. Learning methods based on features extracted from machine learning and learning methods based on artificial neural network models are widely known, so detailed descriptions thereof will be omitted.

Depending on the field to which the present invention is applied, in addition to creating a calibration model by personalizing and learning the continuous biometric information corresponding to the non-invasive blood glucose information, a calibration model can be created by learning the increase and/or decrease pattern of continuous blood glucose information corresponding to the increase and/or decrease pattern of the non-invasive blood glucose information from the non-invasive blood glucose information in a personalized way to the user. The calibration model for the increase and/or decrease pattern simply calibrates whether the blood glucose of a user level is an increasing or decreasing pattern based on non-invasive blood glucose information, and can be more accurate than calibrating the blood glucose value of a user from non-invasive blood glucose information.

Referring again to FIG. 6, when additional non-invasive blood glucose information is acquired from the non-invasive biometric information measurement device after removing the continuous biometric information measurement device from the body (S117), the additional non-invasive blood glucose information is applied to the calibration model to calibrate the non-invasive blood glucose information (S119).

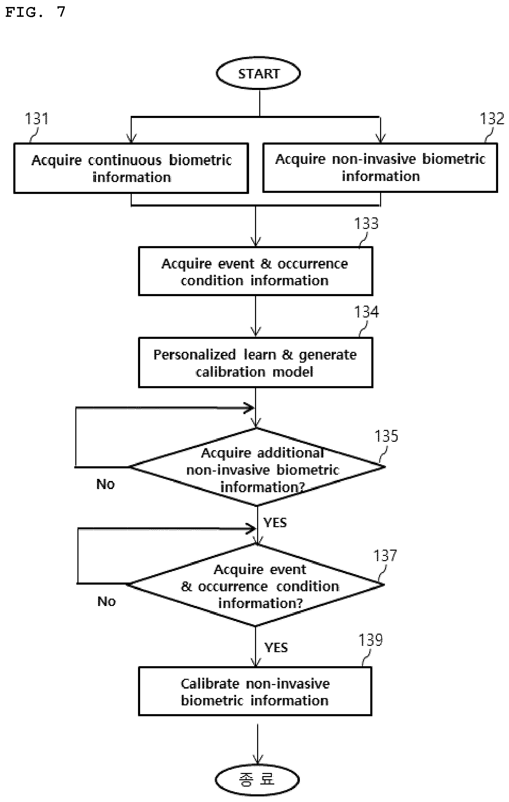

FIG. 7 is a flowchart illustrating a non-invasive biometric information calibration method according to an embodiment of the present invention.

The non-invasive biometric information calibration method according to an embodiment of the present invention described in FIG. 7 relates to a method of personalizing and learning non-invasive blood glucose information by using event information that occurred during a certain period of time or information about event occurrence conditions in addition to the continuous blood glucose information acquired over a certain period of time to determine the non-invasive blood glucose level.

Referring to FIG. 7, when continuous blood glucose information is measured by a continuous biometric information meter for a certain period of time, continuous blood glucose information is received and obtained (S131), and when non-invasive blood glucose information is measured by a non-invasive biometric information measurement device for a certain period of time, non-invasive blood glucose information is acquired (S132).

If an event occurs during a certain period of time, information on the event that occurred and information about event occurrence conditions are acquired (S133). Here, event information is everything that can affect the biometric information of a user, such as exercise performed by the user, type and time of exercise, food consumed, type and amount of food consumed, and degree of stress, and physical conditions (sleep time and quality, presence of illness, etc.).

Meanwhile, when an event occurs, information about event occurrence conditions may include the season in which the event occurred, weather, time, place, location, temperature, humidity, etc.

Such event information and information about event occurrence conditions can be entered directly by the user through the input interface screen, but can be automatically determined through information obtained by various sensors or information obtained by a network.

Continuous blood glucose information measured over a certain period of time, non-invasive blood glucose information measured at the same time as continuous blood glucose information over a certain period of time, and in addition event information and information about event occurrence conditions are applied to the learning model algorithm to provide personalized learning to the user and create a calibration model from the learning results(S134).

By comparing the non-invasive blood glucose information measured from the event information and information about event occurrence conditions with the continuous blood glucose information measured at the same time, the features of the non-invasive blood glucose information are extracted and the continuous blood glucose information corresponding to the extracted features is learned and a calibration model is created, or event information, event occurrence condition information, non-invasive blood glucose information measured and continuous blood glucose information at the same time are applied to the input node of the artificial neural network model to calculate the weight of the hidden node using a linear regression method and a calibration model can be created. Learning methods based on features extracted from machine learning and learning methods based on artificial neural network models are widely known, so detailed descriptions thereof will be omitted.

Depending on the field to which the present invention is applied, in addition to creating a calibration model by personalizing and learning continuous biometric information corresponding to the non-invasive biometric information when an event occurs with event occurrence conditions, a calibration model is created by personalizing and learning the increase and/or decrease pattern of continuous blood glucose information corresponding to the increase and/or decrease pattern of the non-invasive blood glucose information from the non-invasive blood glucose information when the event occurs with event occurrence conditions.

Referring again to FIG. 7, after the continuous biometric information measurement device is removed from a body, additional non-invasive blood glucose information is obtained from the non-invasive biometric information measurement device (S135), and when event information occurring in a user and information about event occurrence conditions are obtained. (S137), non-invasive blood glucose information is corrected by applying additional non-invasive blood glucose information, event information, and information about event occurrence conditions to the calibration model (S139).

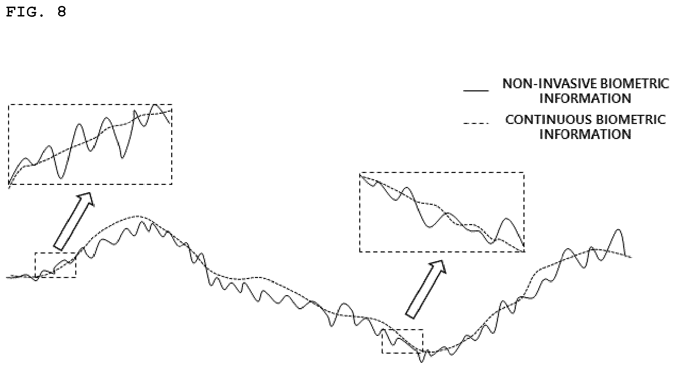

FIG. 8 shows an example of non-invasive blood glucose information measured by a non-invasive biometric information measurement device and continuous blood glucose information measured by a continuous biometric information measurement device.

As shown in FIG. 8(a), because non-invasive blood glucose information measured by a non-invasive biometric information measurement device has various problems, such as the measurement principle of blood glucose information being inaccurate or it being difficult to personalize the non-invasive biometric information measurement device to a user, rather than increasing the same when the actual blood glucose increases or falling the same when the blood glucose falls, meaningless values such as noise that repeat the increase and decrease of the blood glucose value may be obtained.

On the other hand, as shown in FIG. 8(b), continuous blood glucose information measured by a continuous biometric information meter can guarantee a certain degree of accuracy; continuous blood glucose information is measured when the user's blood glucose level increases, it increases equally, and when the blood glucose level drops.

In this way, non-invasive blood glucose information can be personalized to a user and calibrated using continuous blood glucose information measured over a certain period of time by a continuous biometric information measurement device.

This type of continuous biometric information measurement device is inserted and attached to the body of u user for a certain period of time to measure continuous blood glucose information, and non-invasive blood glucose information measured by a non-invasive biometric information measurement device can be personalized to a user and calibrated using continuous blood glucose information obtained continuously over a certain period of time.

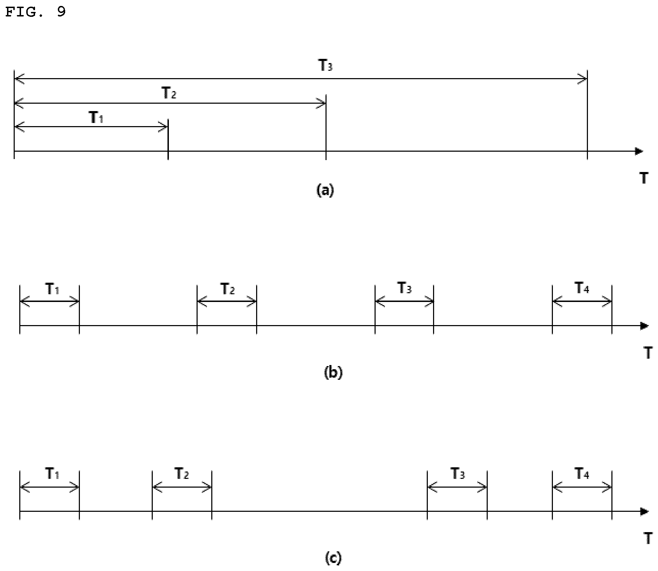

FIG. 9 explains a certain period of time during which a continuous biometric information measurement device is worn on a body.

Referring to FIG. 9(a), the continuous biometric information measurement device can be inserted and attached to the body of a user for a certain period of time among T₁, T₂, T₃.

Here, T₁, T₂, T₃ are the period of use of the biometric information measurement device, which can be worn on the user's body for, for example, 1 week, 15 days, or 1 month.

However, even if the period of use of the continuous biometric information measurement device is one month, it can be worn on a body for T₁, T₂, T₃, which is shorter than the period of use if necessary. Here, the period of measurement of continuous blood glucose information by inserting and wearing the continuous biometric information measurement device on the body can be set to the time when the calibration model is completed. In other words, even if the continuous biometric information measurement device has been used for one month, if a calibration model with the required accuracy is created from continuous blood glucose information measured for 10 days, the continuous biometric information measurement device can be removed after 10 days.

In the present invention, continuous blood glucose information can be measured for a plurality of certain periods of time using a plurality of continuous biometric information measurement devices, and a more accurate calibration model can be created from the plurality of continuous blood glucose information measured over certain periods of time.

A plurality of regular periods for measuring continuous blood glucose information by inserting and attaching a plurality of continuous biometric information measurement devices can be set to be spaced apart from each other. As shown in FIG. 9(b), a plurality of regular periods (T₁, T₂, T₃, T₄) are set to be spaced apart at equal time intervals or, as shown in FIG. 9(c), a plurality of certain periods ((T₁, T₂, T₃, T₄) may be set to be spaced apart from each other at different intervals depending on at least one of environmental conditions, seasonal conditions, user physical conditions, and user physiological conditions.

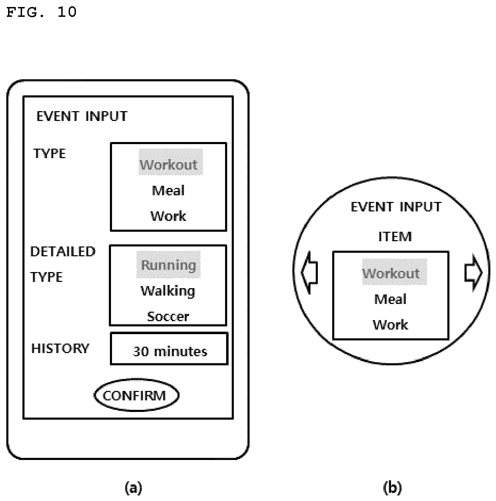

FIG. 10 illustrates an example of an interface screen for inputting event information in the present invention.

As shown in FIG. 10(a), an input interface screen for inputting event information is activated on the display unit of a user terminal, and s user can input the event type, detailed type, event details, etc. on the input interface screen.

As shown in FIG. 10(b), in the case that the non-invasive biometric information measurement device is equipped with a display unit, an input interface screen for inputting event information is activated on the display unit, and a user can input event type, detailed type, event history, etc. on the input interface screen.

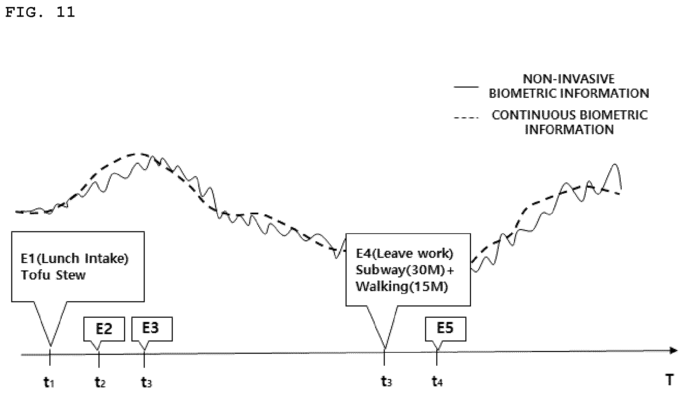

FIG. 11 illustrates an example of event information input through an input interface screen.

As shown in FIG. 11, when events that occurred to the user during a certain period of time and information about occurrence conditions when the events occurred are entered on the input interface screen, an event icon (E) is displayed indicating that event information that occurred over time and information about event occurrence conditions have been entered. When an event icon is selected, detailed information about the event and occurrence condition information are activated.

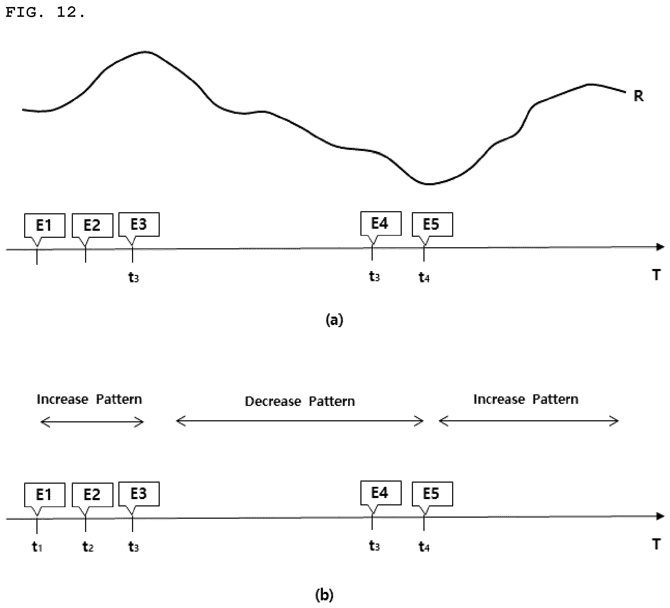

FIG. 12 is a diagram illustrating an example of displaying blood glucose information measured using only a non-invasive biometric information meter after removing the continuous biometric information meter.

After removing the continuous biometric information meter, the user is provided with blood glucose information measured using only the non-invasive biometric information meter, and as shown in FIG. 12(a), calibrated blood glucose information (R) by applying event information entered later, later information about event occurrence conditions, and later non-invasive blood glucose information to the calibration model is displayed.

As shown in FIG. 12(b), information on the increase and/or decrease pattern of blood glucose level determined from the blood glucose information corrected by applying the event information entered later, later information about event occurrence conditions, and later non-invasive blood glucose information to the calibration model is displayed.

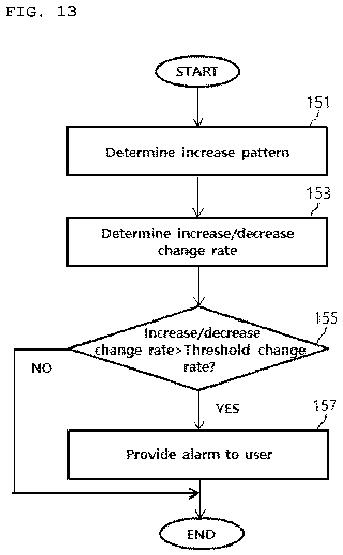

FIG. 13 is a flowchart for explain an example of providing an alarm to a user based on an increase and/or decrease pattern of blood glucose information.

Looking more specifically with reference to FIG. 13, the additional non-invasive blood glucose information, additional event information, and occurrence condition information for the additional event are applied to the calibration model to determine the increase and/or decrease pattern of the blood glucose information (S151), and the increase and/or decrease change rate is determined from the determined increase and/or decrease pattern (S153).

It is determined whether the determined increase and/or decrease change rate is greater than the critical change rate (S155), and if the determined increase and/or decrease change rate is greater than the critical change rate, an alarm message is generated and provided to a user (S157).

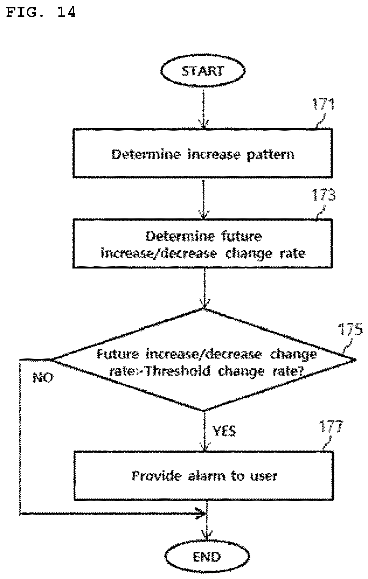

FIG. 14 is a flowchart illustrating an example of providing an alarm to a user based on a subsequent increase and/or decrease change rate of blood glucose information.

Looking more specifically with reference to FIG. 14, the additional non-invasive blood glucose information, additional event information, and additional information about event occurrence conditions are applied to the calibration model to determine the increase and/or decrease pattern of blood glucose information (S171), and based on the determined increase and/or decrease pattern, the expected future increase and/or decrease change rate is determined (S173). Here, the expected future increase and/or decrease change rate can be determined based on the increase and/or decrease pattern expected to be seen in the future from the increase and/or decrease pattern determined based on learning personalized to a user.

It is determined whether the determined future increase and/or decrease change rate is greater than the critical change rate (S175), and if the determined future increase and/or decrease change rate is greater than the critical change rate, an alarm message is generated and provided to a user (S177).

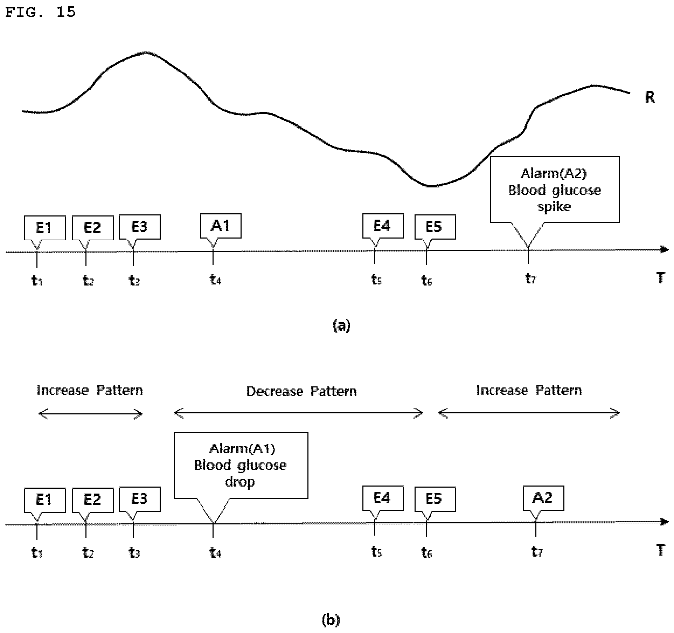

FIG. 15 shows an example of an alarm message provided to a user.

As shown in FIG. 15(a), the event icon (E) indicating the existence of an event that has occurred to a user along with the calibrated blood glucose information (R) and the alarm icon (A) indicating the existence of an alarm message are displayed in the order of time elapse. When selecting the alarm icon (A2), a specific alarm message may be activated.

As shown in FIG. 15(b), an event icon (E) indicating the existence of an event occurring to the user along with Information on increase and/or decrease patterns of blood glucose, and an alarm icon (A) indicating the existence of an alarm message are displayed in the order of time elapse. When selecting the alarm icon (A1), a specific alarm message may be activated.

Meanwhile, the above-described embodiments of the present invention can be written as a program that can be executed on a computer, and can be implemented in a general-purpose digital computer that operates the program using a computer-readable recording medium.

The computer-readable recording media include storage media such as magnetic storage media (e.g., ROM, floppy disk, hard disk, etc.), optical read media (e.g., CD-ROM, DVD, etc.), and carrier waves (e.g., transmission via the Internet).

The present invention has been described with reference to the embodiments shown in the drawings, but these are merely exemplary, and those having ordinary skill in the art will understand that various modifications are possible therefrom. Therefore, the protection scope of the present invention should be determined by the appended claims.

## Claims

1. A method of calibrating non-invasive biometric information, the method comprising:
measuring continuous biometric information of a user by a continuous biometric information measurement device (10) including a sensor configured to be at least partially inserted into a body of the user and to measure the continuous biometric information of the user over a certain period of time;
measuring non-invasive biometric information of the user for the certain period of time using a non-invasive biometric information measurement device (30) that measures the non-invasive biometric information of the user at a distance away from or in contact with skin of the user;
acquiring event information and occurrence condition information about an event occurred to the user while measuring the continuous biometric information;
learning an increase and/or decrease pattern of the continuous biometric information corresponding to an increase and/or decrease pattern of the non-invasive biometric information at each event matching the occurrence condition information using a learning model by comparing the increase and/or decrease pattern of the non-invasive biometric information according to the event information and the occurrence condition information with the increase and/or decrease pattern of the continuous biometric information to generate a calibration model personalized for the user;
acquiring additional non-invasive biometric information of the user, and additional event information and additional occurrence condition information about an additional event occurred to the user after the calibration model personalized for the user is generated; and
calibrating the additional non-invasive biometric information by applying the additional non-invasive biometric information, the additional event information, and the additional occurrence condition information to the personalized calibration model.

2. The method of calibrating the non-invasive biometric information according to claim 1, wherein:
the learned increase and/or decrease pattern of the continuous biometric information corresponding to the increase and/or decrease pattern of the additional non-invasive biometric information is determined to calibrate the increase and/or decrease pattern of the additional non-invasive biometric information.

3. The method of calibrating the non-invasive biometric information according to claim 1 or 2,
wherein the continuous biometric information of the user is measured over multiple certain periods of time by a plurality of continuous biometric information measurement devices (10).

4. The method of calibrating the non-invasive biometric information according to claim 3,
wherein the multiple certain periods are set to be apart.

5. The method of calibrating the non-invasive biometric information according to claim 1,
by determining an increase and/or decrease pattern of the additional non-invasive biometric information and the increase and/or decrease pattern of the continuous biometric information learned to correspond to the additional event information, the increase and/or decrease pattern of the additional non-invasive biometric information is calibrated.

6. The method of calibrating the non-invasive biometric information according to claim 1, further comprising:
determining an increase and/or decrease change rate based on the increase and/or decrease pattern of the calibrated additional non-invasive biometric information; and
when the determined increase and/or decrease change rate exceeds a threshold change rate, providing an alarm to the user.

7. The method of calibrating the non-invasive biometric information according to claim 6, further comprising:
predicting a future increase and/or decrease change rate based on an increase and/or decrease pattern of the calibrated additional non-invasive biometric information; and
when the predicted future increase and/or decrease change rate exceeds a threshold change rate, providing an alarm to the user.

8. The method of calibrating the non-invasive biometric information according to claim 1,
wherein the event information comprises information indicating at least one of exercise performed by the user, a type of the exercise, a time of the exercise, food consumed by the user, a type of the food, an amount of the food, a degree of stress, a sleep time, a sleep quality, and presence of illness.

9. The method of calibrating the non-invasive biometric information according to claim 1,
wherein the occurrence condition information comprises at least one of season information, weather information, time information, place information, location information, temperature information, and humidity information.

## Patentansprüche

1. Verfahren zur Kalibrierung nichtinvasiv gemessener biometrischer Informationen, wobei das Verfahren umfasst:
Messen kontinuierlicher biometrischer Informationen eines Benutzers mittels einer Vorrichtung zur kontinuierlichen Messung biometrischer Informationen (10), die einen Sensor umfasst, der so konfiguriert ist, dass er zumindest teilweise in den Körper des Benutzers eingeführt wird und die kontinuierlichen biometrischen Informationen des Benutzers über einen bestimmten Zeitraum misst;
Messen nichtinvasiv gemessener biometrischer Informationen des Benutzers über den bestimmten Zeitraum mittels einer Vorrichtung zur nichtinvasiven Messung biometrischer Informationen (30), die die nichtinvasiv gemessenen biometrischen Informationen des Benutzers in einem Abstand von der Haut des Benutzers oder in Kontakt mit der Haut des Benutzers misst;
Erfassen von Ereignisinformationen und Informationen über Bedingungen beim Auftreten eines Ereignisses, das bei dem Benutzer während der Messung der kontinuierlichen biometrischen Informationen aufgetreten ist;
Lernen eines Anstiegs- und/oder Abfallmusters der kontinuierlichen biometrischen Informationen, das einem Anstiegs- und/oder Abfallmuster der nichtinvasiv gemessenen biometrischen Informationen bei jedem Ereignis entspricht, das mit den Informationen über die Bedingungen beim Auftreten des Ereignisses übereinstimmt, unter Verwendung eines Lernmodells durch Vergleichen des Anstiegs- und/oder Abfallmusters der nichtinvasiv gemessenen biometrischen Informationen entsprechend den Ereignisinformationen und den Informationen über die Bedingungen beim Auftreten des Ereignisses mit dem Anstiegs- und/oder Abfallmuster der kontinuierlichen biometrischen Informationen, um ein benutzerspezifisch angepasstes Kalibrierungsmodell zu erzeugen;
Erfassen zusätzlicher nichtinvasiv gemessener biometrischer Informationen des Benutzers und zusätzlicher Ereignisinformationen und zusätzlicher Informationen über Bedingungen beim Auftreten eines zusätzlichen Ereignisses, das bei dem Benutzer aufgetreten ist, nachdem das benutzerspezifisch angepasste Kalibrierungsmodell erstellt wurde; und
Kalibrieren der zusätzlichen nichtinvasiv gemessenen biometrischen Informationen durch Anwenden der zusätzlichen nichtinvasiv gemessenen biometrischen Informationen, der zusätzlichen Ereignisinformationen und der zusätzlichen Informationen über die Bedingungen beim Auftreten des Ereignisses auf das benutzerspezifisch angepasste Kalibrierungsmodell.

2. Verfahren zur Kalibrierung der nichtinvasiv gemessenen biometrischen Informationen nach Anspruch 1, wobei:
das gelernte Anstiegs- und/oder Abfallmuster der kontinuierlichen biometrischen Informationen, das dem Anstiegs- und/oder Abfallmuster der zusätzlichen nichtinvasiv gemessenen biometrischen Informationen entspricht, bestimmt wird, um das Anstiegs- und/oder Abfallmuster der zusätzlichen nichtinvasiv gemessenen biometrischen Informationen zu kalibrieren.

3. Verfahren zur Kalibrierung der nichtinvasiv gemessenen biometrischen Informationen nach Anspruch 1 oder 2,
wobei kontinuierliche biometrische Informationen des Benutzers über mehrere bestimmte Zeiträume mittels einer Mehrzahl von Vorrichtungen zur kontinuierlichen Messung biometrischer Informationen (10) gemessen werden.

4. Verfahren zur Kalibrierung der nichtinvasiv gemessenen biometrischen Informationen nach Anspruch 3,
wobei die mehreren bestimmten Zeiträume so festgelegt sind, dass sie voneinander beabstandet sind.

5. Verfahren zur Kalibrierung der nichtinvasiv gemessenen biometrischen Informationen nach Anspruch 1,
wobei durch Bestimmen eines Anstiegs- und/oder Abfallmusters der zusätzlichen nichtinvasiv gemessenen biometrischen Informationen und des Anstiegs- und/oder Abfallmusters der kontinuierlichen biometrischen Informationen, das gelernt wurde, um den zusätzlichen Ereignisinformationen zu entsprechen, das Anstiegs- und/oder Abfallmuster der zusätzlichen nichtinvasiv gemessenen biometrischen Informationen kalibriert wird.

6. Verfahren zur Kalibrierung der nichtinvasiv gemessenen biometrischen Informationen nach Anspruch 1, ferner umfassend:
Bestimmen einer Anstiegs- und/oder Abfalländerungsrate basierend auf dem Anstiegs- und/oder Abfallmuster der kalibrierten zusätzlichen nichtinvasiv gemessenen biometrischen Informationen; und
Ausgeben eines Alarms an den Benutzer, wenn die bestimmte Anstiegs- und/oder Abfalländerungsrate eine Schwellenänderungsrate überschreitet.

7. Verfahren zur Kalibrierung der nichtinvasiv gemessenen biometrischen Informationen nach Anspruch 6, ferner umfassend:
Vorhersagen einer zukünftigen Anstiegs- und/oder Abfalländerungsrate basierend auf einem Anstiegs- und/oder Abfallmuster der kalibrierten zusätzlichen nichtinvasiv gemessenen biometrischen Informationen; und
Ausgeben eines Alarms an den Benutzer, wenn die vorhergesagte zukünftige Anstiegs- und/oder Abfalländerungsrate eine Schwellenänderungsrate überschreitet.

8. Verfahren zur Kalibrierung der nichtinvasiv gemessenen biometrischen Informationen nach Anspruch 1,
wobei die Ereignisinformationen Informationen umfassen, die zumindest eines von einer von dem Benutzer durchgeführten Übung, einer Art der Übung, einem Zeitpunkt der Übung, von dem Benutzer aufgenommenen Nahrung, einer Art der Nahrung, einer Menge der Nahrung, einem Stressgrad, einer Schlafzeit, einer Schlafqualität und dem Vorliegen einer Erkrankung angeben.

9. Verfahren zur Kalibrierung der nichtinvasiv gemessenen biometrischen Informationen nach Anspruch 1,
wobei die Informationen über die Bedingungen beim Auftreten eines Ereignisses zumindest eine von Jahreszeitinformationen, Wetterinformationen, Zeitinformationen, Ortsinformationen, Standortinformationen, Temperaturinformationen und Luftfeuchtigkeitsinformationen umfassen.

## Revendications

1. Procédé d'étalonnage d'informations biométriques non invasives, ledit procédé comprenant :
la mesure d'informations biométriques continues d'un utilisateur par un dispositif de mesure d'informations biométriques continues (10) comprenant un capteur prévu pour être au moins partiellement inséré dans le corps de l'utilisateur et pour mesurer les informations biométriques continues de l'utilisateur pendant une période définie ;
la mesure d'informations biométriques non invasives de l'utilisateur pendant la période définie au moyen d'un dispositif de mesure d'informations biométriques non invasives (30) qui mesure les informations biométriques non invasives de l'utilisateur à distance de la peau de l'utilisateur, ou en contact avec celle-ci ;
l'acquisition d'informations d'événement et d'informations de condition d'occurrence relatives à un événement survenu pour l'utilisateur en mesurant les informations biométriques continues ;
l'apprentissage d'un motif d'augmentation et/ou de diminution des informations biométriques continues correspondant à un motif d'augmentation et/ou de diminution des informations biométriques non invasives pour chaque événement associé aux informations de condition d'occurrence au moyen d'un modèle d'apprentissage, par comparaison du motif d'augmentation et/ou de diminution des informations biométriques non invasives en fonction des informations d'événement et des informations de condition d'occurrence avec le motif d'augmentation et/ou de diminution des informations biométriques continues, afin de générer un modèle d'étalonnage personnalisé pour l'utilisateur ;
l'acquisition d'informations biométriques non invasives additionnelles de l'utilisateur, et d'informations d'événement additionnelles et d'informations de condition d'occurrence additionnelles relatives à un événement additionnel survenu à l'utilisateur après que le modèle d'étalonnage personnalisé pour l'utilisateur a été généré ; et
l'étalonnage des informations biométriques non invasives additionnelles par application des informations biométriques non invasives additionnelles, des informations d'événement additionnelles et des informations de condition d'occurrence additionnelles au modèle d'étalonnage personnalisé.

2. Procédé d'étalonnage d'informations biométriques non invasives selon la revendication 1, où :
le motif d'augmentation et/ou de diminution appris des informations biométriques continues correspondant au motif d'augmentation et/ou de diminution des informations biométriques non invasives additionnelles est déterminé de manière à étalonner le motif d'augmentation et/ou de diminution des informations biométriques non invasives additionnelles.

3. Procédé d'étalonnage d'informations biométriques non invasives selon la revendication 1 ou la revendication 2,
où les informations biométriques continues de l'utilisateur sont mesurées pendant plusieurs périodes définies par une pluralité de dispositifs de mesure d'informations biométriques continues (10).

4. Procédé d'étalonnage d'informations biométriques non invasives selon la revendication 3,
où les plusieurs périodes définies sont prévues séparées.

5. Procédé d'étalonnage d'informations biométriques non invasives selon la revendication 1,
où, par détermination d'un motif d'augmentation et/ou de diminution des informations biométriques non invasives additionnelles et d'un motif d'augmentation et/ou de diminution appris des informations biométriques continues de manière à correspondre aux informations d'événement additionnelles, le motif d'augmentation et/ou de diminution des informations biométriques non invasives additionnelles est étalonné.

6. Procédé d'étalonnage d'informations biométriques non invasives selon la revendication 1, comprenant en outre :
la détermination d'un taux de variation d'augmentation et/ou de diminution sur la base du motif d'augmentation et/ou de diminution étalonné des informations biométriques non invasives additionnelles ; et,
si le taux de variation d'augmentation et/ou de diminution déterminé dépasse un taux de variation seuil, l'émission d'une alarme pour l'utilisateur.

7. Procédé d'étalonnage d'informations biométriques non invasives selon la revendication 6, comprenant en outre :
la prévision d'un futur taux de variation d'augmentation et/ou de diminution sur la base d'un motif d'augmentation et/ou de diminution des informations biométriques non invasives additionnelles étalonnées ; et,
si le futur taux de variation d'augmentation et/ou de diminution prévu dépasse un taux de variation seuil, l'émission d'une alarme pour l'utilisateur.

8. Procédé d'étalonnage d'informations biométriques non invasives selon la revendication 1,
où les informations d'événement comprennent des informations indiquant un exercice effectué par l'utilisateur, et/ou le type d'exercice, et/ou le moment de l'exercice, et/ou un aliment consommé par l'utilisateur, et/ou le type d'aliment, et/ou une quantité d'aliment, et/ou un niveau de stress, et/ou un temps de sommeil, et/ou la qualité du sommeil, et/ou l'existence d'une pathologie.

9. Procédé d'étalonnage d'informations biométriques non invasives selon la revendication 1,
où les informations de condition d'occurrence comprennent au moins une information parmi des informations de saison, des informations météorologiques, des informations temporelles, des informations de lieu, des informations de localisation, des informations de température et des informations d'humidité.
